# EUROPÄISCHE PATENTSCHRIFT

(11) **EP 0 764 696 B1**
(45) Veröffentlichungstag und Bekanntmachung des Hinweises auf die Patenterteilung: **15.05.2002**
(21) Anmeldenummer: 96810602.1
(22) Anmeldetag: 11.09.1996
(51) Int. Cl.: C09B 67/22, C09B 67/48, C08K 5/3415

(54) **Herstellung von Mischkristallen und festen Lösungen von 1,4-Diketopyrrolopyrrolen**
Manufacture of mixed crystals and solid solutions of 1,4-diketopyrrolopyrroles
Fabrication de cristaux mixtes et solutions solides de 1,4-dicétopyrrolopyrroles

(30) Priorität: 20.09.1995 CH 265195
(43) Veröffentlichungstag der Anmeldung: 26.03.1997
(73) Patentinhaber: Ciba Specialty Chemicals Holding Inc., 4057 Basel (CH)
(72) Erfinder: Mizuguchi, Jin, 231 Yokohama (JP); Hao, Zhimin, 1723 Marly (CH); Wallquist, Olof, 1723 Marly (CH); Iqbal, Abul, 1732 Arconciel (CH)

(56) Entgegenhaltungen:
- EP-A- 0 256 983
- EP-A- 0 348 889
- EP-A- 0 654 506
- EP-A- 0 704 497
- WO-A-90/01480

## Beschreibung

Die vorliegende Erfindung betrifft die Herstellung von Mischkristallen und festen Lösungen aus zwei unterschiedlichen symmetrischen 1,4-Diketopyrrolopyrrolen durch Erhitzen eines entsprechenden Gemisches auf höhere Temperaturen.

Aus US-Patent 4 783 540 ist bekannt, dass beim Mischen zweier verschiedener 1,4-Diketopyrrolopyrrole, vorzugsweise in einem Verhältnis von 65-90:35-10 Gew.%, und nachfolgende Behandlung,
- durch Kontaktierung in polaren organischen Lösungsmitteln, bevorzugt durch Verrühren der Komponentenmischung bei Rückflusstemperatur,
- durch alkalische Umfällung der Komponentenmischung in polaren organischen Lösungsmitteln oder durch Verrühren der Komponentenmischung in polaren organischen Lösungsmitteln in Gegenwart von Alkalialkoholaten, Alkalihydroxiden oder quatemären Ammoniumverbindungen,
- durch saure Umfällung, d.h. Auflösung der Komponentenmischung in Säure und Fällung der festen Lösung durch Verdünnen mit Wasser oder
- durch intensive Mahlung oder Knetung der Komponentenmischung, gegebenenfalls mit anschliessender Rekristallisation in Wasser und/oder organischen Lösungsmitteln,
feste Lösungen erhalten werden können. Die festen Lösungen sind dabei durch ihre Röntgenbeugungsdiagramme charakterisiert, wobei sich die Röntgenbeugungsdiagramme der festen Lösungen von der Summe der Röntgenbeugungsdiagramme der Einzelkomponenten unterscheiden.

EP-A-0 348 889 offenbart Mischkristalle bestehend aus zwei Phthalocyaninen, welche durch simultane Sublimation der Komponenten, einzeln oder gemischt, erhalten werden können. Eine Temperatur von zirka 450 bis 500°C wird angegeben.

Es ist nun gefunden worden, dass durch einfaches Erhitzen eines Gemisches zweier unterschiedlicher 1,4-Diketopyrrolopyrrole in fester Form ganz überraschend Mischkristalle oder feste Lösungen entstehen.

Um Missverständnisse bezüglich der Definition von festen Lösungen und Mischkristallen zu vermeiden, wird hier noch festgehalten, dass das erfindungsgemässe Verfahren, je nach Struktur und Mischungsverhältnis der Komponenten, zwei Arten von Produkten liefern kann:
- Feste Lösungen des "Wirt-Gast"-Typs, wobei die "Gasr"-Komponente sich in das Kristallgitter des "Wirts" einlagert. Das Röntgenbeugungsdiagramm solcher festen Lösungen enthält die Linien der "Wirt"-Komponente. Erscheinen im Röntgenbeugungsdiagramm keine Linien der "Gast"-Komponente, so ist das Produkt kristallographisch rein, d.h. es handelt sich um eine einphasige feste Lösung;
- Mischkristalle, bei denen ein völlig neues Kristallgitter gebildet wird. Das Röntgenbeugungsdiagramm des Mischkristalls unterscheidet sich von den Röntgenbeugungsdiagrammen der Einzelkomponenten.

Die vorliegende Erfindung betrifft demnach ein Verfahren zur Herstellung von Mischkristallen oder festen Lösungen von 1,4-Diketopyrrolo[3,4-c]pyrrolen, bestehend aus zwei unterschiedlichen Verbindungen der Formel 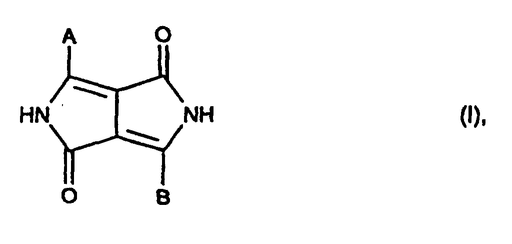 worin A und B, unabhängig voneinander für eine Gruppe der Formel 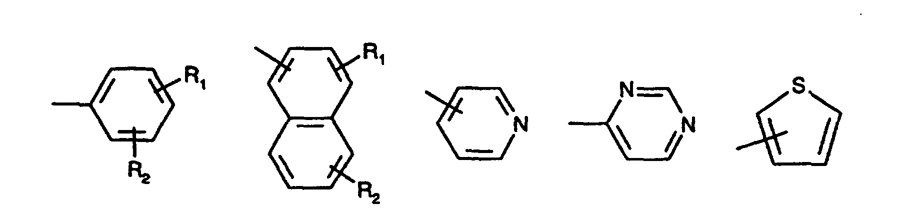 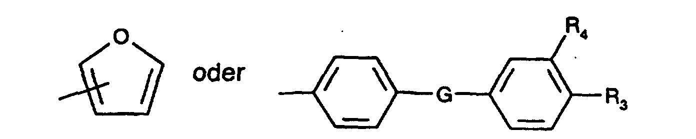 stehen, worin
R₁ und R₂ unabhängig voneinander Wasserstoff, Halogen, C₁-C₁₈-Alkyl, C₁-C₁₈-Alkoxy, C₁-C₁₈-Alkylmercapto, C₁-C₁₈-Alkylamino, C₂-C₁₈-Alkoxycarbonyl, C₂-C₁₈-Alkylaminocarbonyl,-CN, -NO₂, Phenyl, Trifluormethyl, C₅-C₆-Cycloalkyl, -C=N-(C₁-C₁₆-Alkyl). 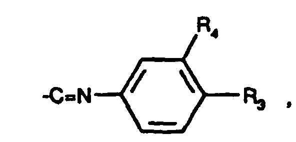 Imidazolyl, Pyrrazolyl, Triazolyl, Piperazinyl, Pyrrolyl, Oxazolyl, Benzoxazolyl, Benzthiazolyl, Benzimidazolyl, Morpholinyl, Piperidinyl oder Pyrrolidinyl bedeuten,
G -CH₂-, -CH(CH₃)-, -C(CH₃)₂-, -CH=N-, -N=N-, -O-, -S-, -SO-, -SO₂-, -CONH- oder -NR₇- ist, R₃ und R₄ unabhängig voneinander Wasserstoff, Halogen, C₁-C₆-Alkyl, C₁-C₁₈Alkoxy oder - CN sind, R₅ und R₆ unabhängig voneinander Wasserstoff, Halogen oder C₁-C₆-Alkyl und R₇ Wasserstoff oder C₁-C₆-Alkyl bedeuten, dadurch gekennzeichnet, dass ein Gemisch zweier unterschiedlicher Verbindungen der Formel I im Mol verhältnis von 50:50 bis 95:5 in fester Form auf Temperaturen zwischen 220 und 380°C, bevorzugt zwischen 240 und 360°C, insbesondere zwischen 270 und 340°C erhitzt wird.

Bedeuten etwaige Substitutenten Halogen, dann handelt es sich z.B. um Jod, Fluor, insbesondere Brom und bevorzugt Chlor;
bei C₁-C₆-Alkyl handelt es sich beispielsweise um Methyl, Ethyl, n-Propyl, Isopropyl, n-Butyl, sec.-Butyl, tert-Butyl, n-Amyl, tert.-Amyl, Hexyl und bei C₁-C₁₈-Alkyl zusätzlich z.B. um Heptyl, Octyl, 2-Ethylhexyl, Nonyl, Decyl, Dodecyl, Tetradecyl, Hexadecyl oder Octadecyl;
C₁-C₁₈-Alkoxy bedeutet, auch in C₂-C₁₈-Alkoxycarbonyl, z.B. Methoxy, Ethoxy, n-Propoxy, lsopropoxy, Butyloxy, Hexyloxy, Decyloxy, Dodecyloxy, Hexadecyloxy oder Octadecyloxy;
C₁-C₁₈-Alkylmercapto steht beispielsweise für Methylmercapto, Ethylmercapto, Propylmercapto, Butylmercapto, Octylmercapto, Decylmercapto, Hexadecylmercapto oder Octadecylmercapto;
C₁-C₁₈-Alkylamino bedeutet, auch in C₂-C₁₈-Alkylaminocarbonyl, z.B. Methylamino, Ethylamino, Propylamino, Hexylamino, Decylamino, Hexadecylamino oder Octadecylamino.
C₅-C₆-Cycloalkyl steht z.B. für Cyclopentyl und insbesondere für Cyclohexyl.

Von besonderem Interesse ist das erfindungsgemässe Verfahren zur Herstellung von Mischkristallen, bei denen in Formel I A und B unabhängig voneinander eine Gruppe der Formel 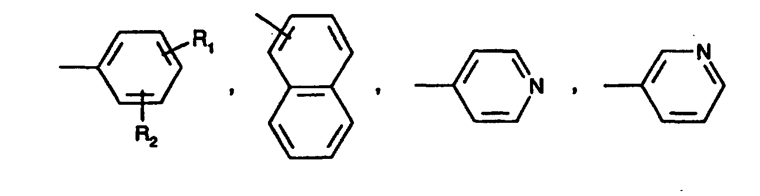 oder 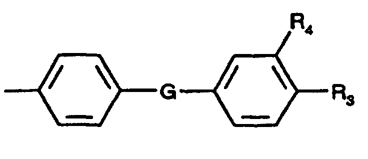 sind,
worin R₁ und R₂ unabhängig voneinander Wasserstoff, Chlor, Brom, C₁-C₄-Alkyl, C₁-C₆-Alkoxy, C₁-C₆-Alkylamino, Phenyl oder CN bedeuten,
G -O-, -NR₇-, -N=N- oder -SO₂- ist,
R₃ und R₄ Wasserstoff und R₇ Wasserstoff, Methyl oder Ethyl bedeuten,
und insbesondere von jenen, worin in Formel I A und B gleich sind und eine Gruppe der Formel 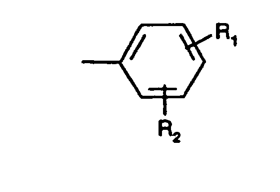 darstellen, worin R₁ und R₂ unabhängig voneinander Wasserstoff, Methyl, tert.-Butyl, Chlor, Brom, Phenyl oder CN bedeuten. R₂ ist bevorzugt Wasserstoff.

Die beiden unterschiedlichen Komponenten der Formel I liegen zweckmässig im Molverhältnis von 50-95 % zu 50-5 %, bevorzugt 50-60 % zu 50-40 % zueinander vor. Dies führt zu Mischkristallen bzw. festen Lösungen mit interessanten Nuancenverschiebungen im Vergleich zu den Ausgangsprodukten.

Einer der beiden Komponenten kann selbst ein Gemisch zweier unterschiedlicher Verbindungen der Formel I sein.

Die erfindungsgemässe Herstellung von Mischkristallen erfolgt zweckmässig durch inniges Vermischen der oben definierten unterschiedlichen Komponenten der Formel I nach allgemein bekannten Methoden und
- Aufheizen der Komponentenmischung auf die wie oben erwähnt erforderliche Temperatur, z.B. in einem Ofen
   oder
- Sublimation der Komponentenmischung in einem Sublimiergerät (z.B. wie in J.Mizuguchi, Crystal Research and Technology, 16, 695-700 (1981) beschrieben).

Im letzteren Falle bilden sich die reinen Mischkristalle beim Kondensieren aus der Gasphase. Es muss allerdings geachtet werden, dass eine hohe Temperatur, leicht niedriger als der Sublimationspunkt der Ausgangssubstanzen, über eine längere Kondensationszone konstant erhalten wird. Diese längere Kondensationszone erlaubt es die Mischkristalle von unreagiertem Ausgangsmaterial zu trennen, das bei tieferen Temperaturen als die Mischkristalle kondensiert.

Eine weitere Ausführungsform besteht darin, dass die beiden unterschiedlichen Komponenten einzeln abwechselnd in zwei oder mehreren dünnen Schichten im Hochvakuum auf ein geeignetes Substrat (z.B. Glas oder hitzebeständige Polymere) aufgedampft und mit einer Photopolymer-Schutzschicht (z.B. UV-vemetzbarer Lack auf Acrylbasis ®DAICURE CLEAR SD-17 (DIC GmbH); UV-Lack auf Acrylbasis ®RENGOLUX Rz 3203/001 farblos (Dr. Renger GmbH)) überzogen werden und nachfolgend einer Laserbestrahlung, z.B. mittels Ar⁺-Laser (λ=514nm), unterzogen werden.

Die Mischkristallbildung erfolgt dabei im festen Zustand.

Letztere Methode eignet sich ausgezeichnet für die Anwendung in optischen Aufzeichnungsverfahren.

Zur Erleichterung der Vermischung der beiden Komponenten kann ein Mischungshilfsmittel, wie z.B. NaCl oder bevorzugt NaF, zugegeben werden. Zweckmässig wird das Mischungshilfsmittel in Mengen von 0 bis 20, vorzugsweise 0,5 bis 2 Gew%, bezogen auf die Komponentenmischung zugegeben.

Zur Optimierung der Pigmenteigenschaften kann es zweckmässig sein, die Pigmente nachzubehandeln. Die Rekristallisation bzw. thermische Behandlung geschieht nach für Pigmente üblichen Methoden. Im allgemeinen handelt es sich um eine thermische Nachbehandlung in Wasser oder in einem organischen Lösungsmittel, gegebenenfalls unter Druck. Vorzugsweise verwendet man organische Lösungsmittel, z.B. durch Halogenatome, Alkyl- oder Nitrogruppen substituierte Benzole, wie Xylole, Chlorbenzol, o-Dichlorbenzol oder Nitrobenzol, sowie Pyridinbasen, wie Pyridin, Picolin oder Chinolin, ferner Ketone, wie Cyclohexanon, Alkohole, wie Isopropanol, Butanole oder Pentanole, Ether, wie Ethylenglykolmonomethyl- oder -monoethylether, Amide, wie Dimethylformamid oder N-Methylpyrrolidon, sowie Dimethylsulfoxid oder Sulfolan. Man kann die Nachbehandlung auch in Wasser, gegebenenfalls unter Druck, in Gegenwart von organischen Lösungsmitteln und/oder mit Zusatz von oberflächenaktiven Substanzen durchführen.

Sowohl die Mischkristalle als auch die festen Lösungen, die nach dem erfindungsgemässen Verfahren hergestellt werden, können als Pigmente zum Färben von hochmolekularem organischem Material verwendet werden.

Hochmolekulare organische Materialien, die mit den erfindungsgemäss erhaltenen Mischkristallen oder festen Lösungen gefärbt bzw. pigmentiert werden können, sind z.B. Celluloseether und -ester, wie Ethylcellulose, Nitrocellulose, Celluloseacetat, Cellulosebutyrat, natürliche Harze oder Kunstharze, wie Polymerisationsharze oder Kondensationsharze, wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, ABS, Polyphenylenoxide, Gummi, Casein, Silikon und Silikonharze, einzeln oder in Mischungen.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemäss hergestellten Mischkristalle oder festen Lösungen als Toner oder in Form von Präparaten einzusetzen.

Bezogen auf das zu pigmentierende hochmolekulare organische Material kann man die erfindungsgemäss hergestellten Mischkristalle oder festen Lösungen in einer Menge von 0,01 bis 30 Gew.%, vorzugsweise von 0,1 bis 10 Gew. %, einsetzen.

Die Pigmentierung der hochmolekularen organischen Substanzen mit den erfindungsgemäss hergestellten Mischkristallen oder festen Lösungen erfolgt beispielsweise derart, dass man solche Mischkristalle bzw. festen Lösungen gegebenenfalls in Form von Masterbatches diesen Substraten unter Verwendung von Walzwerken, Misch- oder Mahlapparaten zumischt. Das pigmentierte Material wird hierauf nach an sich bekannten Verfahren, wie Kalandrieren, Pressen, Strangpressen, Streichen, Giessen oder Spritzgiessen, in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können vor oder nach der Einverleibung der erfindungsgemäss hergestellten Mischkristalle bzw. festen Lösungen in die Polymeren eingearbeitet werden. Zwecks Erzielung verschiedener Farbtöne ist es femer möglich, den hochmolekularen organischen Stoffen neben den erfindungsgemäss hergestellten Mischkristallen bzw. festen Lösungen noch Füllstoffe bzw. andere farbgebende Bestandteile, wie Weiss-, Bunt- oder Schwarzpigmente, in beliebigen Mengen zuzufügen.

Zum Pigmentieren von Lacken, Anstrichstoffen und Druckfarben werden die hochmolekularen organischen Materialien und die erfindungsgemäss hergestellten Mischkristalle oder festen Lösungen gegebenenfalls zusammen mit Zusatzstoffen, wie Füllmitteln, anderen Pigmenten, Siccativen oder Weichmachem, in einem gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

Besonders geeignet sind die erfindungsgemäss hergestellten Mischkristalle und festen Lösungen zum Einfärben von Kunststoffen, insbesondere Polyvinylchlorid und Polyolefinen, und Lacken, insbesondere Automobillacken.

Mischkristalle bzw. feste Lösungen können aber auch im Polymer selbst gebildet werden, wenn man die zwei Pyrrolopyrrol-Komponenten einzeln in das Polymer einarbeitet und das ganze auf die geeignete Temperatur erhitzt. Eine weitere Ausführungsform der Erfindung besteht also darin, dass man zwei unterschiedliche Verbindungen der Formel I zusammen mit einem Polymer in einem Extruder bei Temperaturen von 160 bis 210°C dispergiert und anschliessend bei 220 bis 300°C zu Formen verspritzt. Man erhält so Kunststoffformen, die mit den sich im Extruder gebildeten Mischkristallen (oder feste Lösungen) gefärbt sind. Die Mischkristallbildung, findet im allgemeinen im Polymer bei Temperaturen von mindestens 220 bis 300°C statt.
Dazu geeignete Polymere sind z.B. Polycarbonate, Polyolefine, Polystyrol, Polyamide, Polyester, ABS oder Polyphenylenoxide.

In Färbungen, beispielsweise von Polyvinylchlorid oder Polyolefinen, zeichnen sich sowohl die Mischkristalle als auch die festen Lösungen, die nach dem erfindungsgemässen Verfahren hergestellt werden, durch gute allgemeine Pigmenteigenschaften, wie gute Dispergierbarkeit, hohe Farbstärke und Reinheit, gute Migrations-, Hitze-, Licht- und Wetterbeständigkeit sowie gute Deckkraft aus.

Die nachfolgenden Beispiele erläutem die Erfindung.

Beispiel 1: 2,36 g (6,6 mMol) des Pyrrolopyrrols der Formel 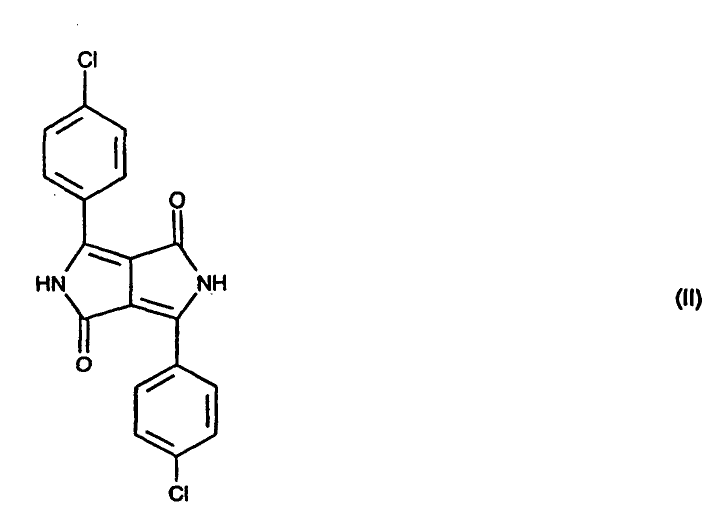 2,09 g (6,6 mMol) des Pyrrolopyrrols der Formel 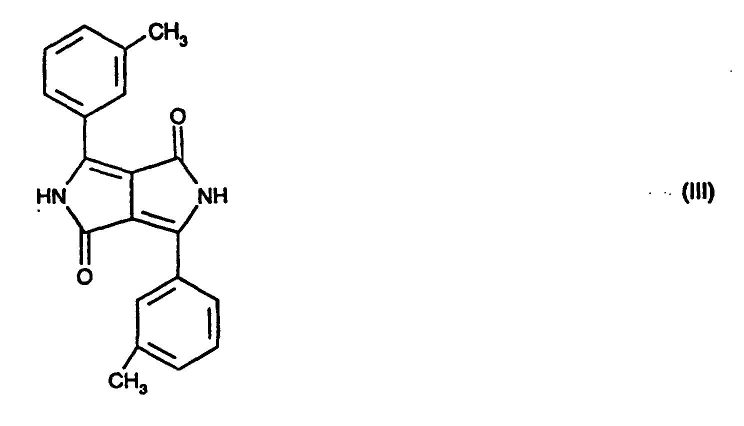 und 40 mg Natriumfluorid werden in einem Mörser innig vermischt. Das erhaltene Gemisch in Pulverform wird in einer mit einem Deckel verschlossenen Porzellanschale 4 Stunden in einem Ofen auf 300°C erhitzt. Man erhält ein rotes pulverförmiges Produkt in quantitativer Ausbeute, welches gründlich mit Wasser gewaschen und im Vakuumtrockenschrank bei 80°C getrocknet wird.

| Analyse | C | H | N | Cl |
|---|---|---|---|---|
| ber. | 67,76% | 3,89% | 8,32% | 10,53% |
| gef. | 67,19% | 3,86% | 8,27% | 10,51% |

Das Röntgenbeugungsdiagramm ist durch folgende Beugungslinien

| Netzebenenabstände (d-Werte in Å) | doppelte Glanzwinkel (2Θ) | relative Intensität |
|---|---|---|
| 15.1550 | 5.83 | 100 |
| 6.8411 | 12.93 | 29 |
| 6.4135 | 13.80 | 24 |
| 6.0442 | 14.64 | 41 |
| 5.0401 | 17.58 | 18 |
| 3.7190 | 23.91 | 24 |
| 3.6148 | 24.61 | 14 |
| 3.3203 | 26.83 | 86 |
| 3.1550 | 28.26 | 27 |
| 2.8766 | 31.06 | 14 |
| 2.7846 | 32.12 | 9 |

gekennzeichnet

Beispiel 2: 0,9 g (2,5 mMol) des Pyrrolopyrrols der Formel II (vgl. Beispiel 1), 1,0 g (2,2 mMol) des Pyrrolopyrrols der Formel 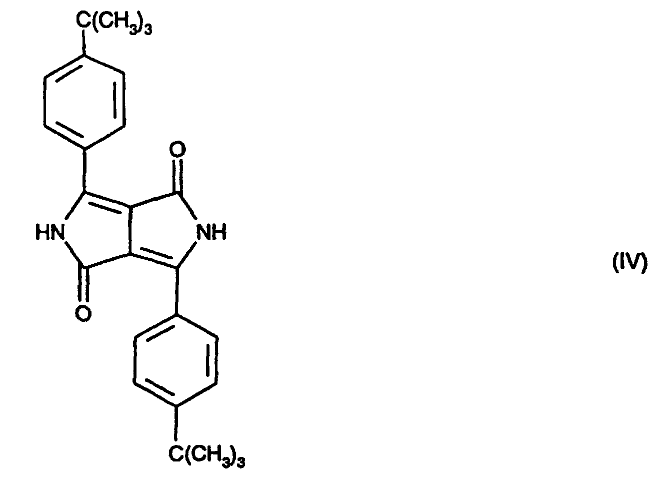 und 10 mg Natriumfluorid werden in einem Mörser innig vermischt. Das erhaltene Gemisch in Pulverform wird in einer mit einem Deckel verschlossenen Porzellanschale 1 Stunde in einem Ofen auf 270°C erhitzt. Man erhält ein blaurotes pulverförmiges Produkt in quantitativer Ausbeute, das gründlich mit Wasser gewaschen und im Vakuumtrockenschrank bei 80°C getrocknet wird.

| Analyse | C | H | N | Cl |
|---|---|---|---|---|
| ber. | 69,25% | 4,94% | 7,42% | 9,93% |
| gef. | 69,15% | 5,10% | 7,23% | 9,54% |

Das Röntgenbeugungsdiagramm ist durch folgende Beugungslinien

| Netzebenenabstände (d-Werte in Å) | doppelte Glanzwinkel (2Θ) | relative Intensität |
|---|---|---|
| 19.4419 | 4.54 | 100 |
| 9.5816 | 9.22 | 8 |
| 6.3592 | 13.52 | 23 |
| 4.9987 | 17.73 | 64 |
| 4.8998 | 18.09 | 37 |
| 3.7966 | 23.41 | 9 |
| 3.6459 | 24.40 | 10 |
| 3.3709 | 26.42 | 56 |
| 3.2363 | 27.54 | 18 |
| 3.1617 | 28.20 | 11 |
| 3.0412 | 29.34 | 10 |

gekennzeichnet.

Beispiel 3: 5,36 g (15 mMol) des Pyrrolopyrrols der Formel II (vgl. Beispiel 1), 4,01 g (10 mMol) des Pyrrolopyrrols der Formel IV (vgl. Beispiel 2) werden in einem Mörser innig vermischt. Das erhaltene Gemisch in Pulverform wird in einer mit einem Deckel verschlossenen Porzellanschale 4 Stunden in einem Ofen auf 300°C erhitzt. Man erhält ein blaurotes pulverförmiges Produkt in quantitativer Ausbeute.

| Analyse | C | H | N | Cl |
|---|---|---|---|---|
| ber. | 67,51% | 4,51% | 7,51% | 11,91% |
| gef. | 67,53% | 4,62% | 7,24% | 11,31% |

Das Röntgenbeugungsdiagramm ist durch folgende Beugungslinien

| Netzebenenabstände (d-Werte in Å) | doppelte Glanzwinkel (2Θ) | relative Intensität |
|---|---|---|
| 19.1018 | 4.62 | 100 |
| 15.8733 | 5.56 | 8 |
| 6.3224 | 14.00 | 20 |
| 4.9820 | 17.79 | 42 |
| 4.8891 | 18.13 | 27 |
| 3.7810 | 23.51 | 9 |
| 3.6309 | 24.50 | 9 |
| 3.4538 | 25.77 | 9 |
| 3.3589 | 26.52 | 39 |
| 3.2236 | 27.65 | 14 |
| 3.1532 | 29.34 | 18 |
| 3.0371 | 29.39 | 8 |

gekennzeichnet.

Beispiel 4:
7,5g des Mischkristalls aus Beispiel 1,98,9g CAB-Lösung bestehend aus 41,0 g Celluloseacetobutyrat (CAB 531.1, 20%ig in Butanol/Xylol 2:1 Eastman Chem.)
1,5 g Zirkonium Octoat,
18,5 g ®SOLVESSO 150* (ESSO),
21,5 g Butylacetat und
17,5 g Xylol,
36,5 g Polyesterharz ®DYNAPOL H700 (Dynamit Nobel), 4,6 g Melaminharz MAPRENAL MF650 (Hoechst) und 2,5 g Dispergiermittel ®DISPERBYK160 (Byk Chemie) werden zusammen während 90 Minuten mit einer Schüttelmaschine dispergiert (Total Lack 150g; 5% Pigment). 27,69 g des so erhaltenen Volltonlacks werden für die Base-coat-Lackierung mit 17,31g Al-Stammlösung (8%ig) bestehend aus

| | |
|---|---|
| 12,65 g | ®SILBERLINE SS 3334AR, 60%ig (Silberline Ltd.) |
| 56,33 g | CAB Lösung (Zusammensetzung wie oben) |
| 20,81 g | Polyesterharz ®DYNAPOL H700 |
| 2,60 g | Melaminharz ®MAPRENAL MF650 |
| 7,59 g | ®SOLVESSO 150 |

gemischt und auf ein Aluminiumblech spritzappliziert (Nassfilm ca.20µm). Nach einer Abdunstzeit von 30 Minuten bei Raumtemperatur wird ein TSA-Lack bestehend aus

| | |
|---|---|
| 29,60 g | Acrylharz ®URACRON 2263 XB, 50%ig in Xylol/Butanol (Chem. Fabrik Schweizerhalle), |
| 5,80 g | Melaminharz ®CYMEL 327, 90%ig in Isobutanol, |
| 2,75 g | Butylglycolacetat, |
| 5,70 g | Xylol, |
| 1,65 g | n-Butanol |
| 0,50 g | Siliconöl, 1%ig in Xylol, |
| 3,00 g | Lichtschutzmittel ®TINUVIN 900, 10%ig in Xylol (Ciba) |
| 1,00 g | Lichtschutzmittel ®TINUVIN 292, 10%ig in Xylol (Ciba) |

als Top-coat-Lackierung spritzappliziert (Nassfilm ca.50µm). Anschliessend wird der Lack nach weiteren 30Minuten Abdunsten bei Raumtemperatur, 30Minuten bei 130°C eingebrannt.
*SOLVESSO: Aromatische Kohlenwasserstoffe

Beispiel 5: 0,6 g des Mischkristalls von Beispiel 1 werden mit 67g Polyvinylchlorid, 33 g Dioctylphthalat, 2 g Dibutylzinndilaurat und 2 g Titandioxid vermischt und auf einem Walzenstuhl während 15 Minuten bei 160°C zu einer dünnen Folie verarbeitet. Die so erzeugte rote PVC-Folie ist sehr farbstark, migrations- und lichtbeständig.

Beispiel 6: 1000g Polypropylengranulat (®DAPLEN PT-55, Chemie LINZ) und 20 g eines 50%-igen Pigmentpräparates, bestehend aus 10 g der festen Lösung von Beispiel 3 und 10 Mg-Behenat, werden in einer Mischtrommel intensiv vermischt. Das so behandelte Granulat wird bei 260 bis 285°C nach dem Schmelzspinnverfahren versponnen. Man erhält rotgefärbte Fasern mit sehr guten Licht- und textilen Echtheiten.

Beispiel 7: 0,5g (1,7mMol) Pyrrolopyrrol der Formel 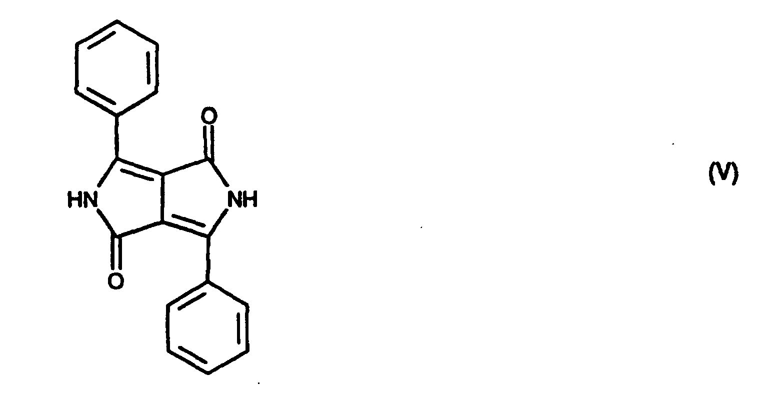 und 0,7g (1,7mMol) Pyrrolopyrrol der Formel IV (vgl. Beispiel 2) werden vermischt und in ein Aufdampfungsschiffchen aus Tantal gegeben. Das Schiffchen wird dann in eine Sublimationsröhre eingeführt; die anschliessend mittels einer Drehschieberpumpe, welche mit Kühlfallen ausgestattet ist, ausgepumpt wird. In die Sublimationsröhre wird danach Ar-Trägergas bei einer Ar-Strommenge von 0,15 ml/Min eingeleitet und die Sublimation wird bei 340°C während 24 Stunden durchgeführt. Eine dunkelrote Substanz kondensiert in der Temperaturzone von 310°C in Form eines kristallinen Pulvers (1,08 g, 90% d.Th.). Nach spektroskopischer und kristallographischer Analyse erweist sich das Produkt als praktisch identisch mit dem Produkt von Beispiel 1. Die unreagierten Ausgangsprodukte kondensieren in einer niedrigeren Temperaturzone und können so leicht abgetrennt werden.

Beispiel 8: Eine Aufdampfungsapparatur, die mit zwei unabhängig kontrollierbaren Aufdampfschiffchen ausgestattet ist, wird zur Herstellung von Multischichten verwendet. Eine erste dünne Schicht Pyrrolopyrrol der Formel V (vgl. Beispiel 7) (150Å) und eine zweite Schicht Pyrrolopyrrol der Formel IV (vgl. Beispiel 2) (ebenfalls 150 Å), wobei die beiden Produkte im Molverhältnis 1:1 eingesetzt werden, werden sukzessive unter Hochvakuum auf eine Glasplatte aufgedampft. Das Prozedere wird stets unter Vakuum dreimal wiederholt, so dass alternierend vier Schichtenpaare auf der Glasplatte aufgedampft werden. Danach wird eine Schutzschicht (Schichtdicke ca. 10µm) aus einem UVvernetzbaren Lack auf Acrylbasis (®DAICURE CLEAR SD-17; DIC GmbH) aufgetragen und mit UV-Licht vernetzt. Die so beschichtete Glasplatte wird dann mit Ar⁺-Laser (λ=514 nm; 400 mW) unter einer Scanninggeschwindigkeit von 100mm/Sek. belichtet. Die Belichtung bewirkt eine sofortige Nuancenverschiebung von Rot nach Dunkelrot. Das Absorptionsspektrum mit zwei Peaks bei 494 und 576 nm entspricht im wesentlichen demjenigen des Mischkristalls von Beispiel 1.

Dieses Multischicht-System eignet sich ausgezeichnet für die Anwendung in einmal beschreibbare optische Platten.

Beispiel 9: Eine Mischung von 0,5 g des Pyrrolopyrrols der Formel IV (vgl. Beispiel 2) und 0.5 g des Pyrrolopyrrols der Formel V (vgl. Beispiel 7), 1,0 g Antioxidans (®IRGANOX) 1010, CIBA-GEIGY AG) und 1000 g Polyethylen-HD Granulat (®VESTOLEN 60-16, HUELS) wird während 15 Minuten in einer Glasflasche auf einer Rollbank vorgemischt. Danach wird die Mischung in zwei Passagen auf einem Einwellenextruder bei Temperaturen von 160 bis 200 C extrudiert. Das so erhaltene Granulat wird auf der Spritzgussmaschine (®FERROMATIK AARBURG 200) in 5 Minuten bei 240 C zu Platten verspritzt.

Man erhält dunkelrote Platten. Die Farbe ist die gleiche, wie diejenige der mit dem entsprechenden Mischkristall eingefärbten Polyethylenplatte.

Beispiel 10: Man verfährt wie in Beispiel 9, ersetzt aber das Pyrrolopyrrol der Formel V mit der gleichen Menge des Pyrrolopyrrols der Formel II (vgl. Beispiel 1). Man erhält dunkelrote Platten. Die Farbe entspricht genau derjenigen, der mit dem entsprechenden Mischkristall pigmentierten Polyethylenplatte.

Beispiel 11: Man verfährt wie in Beispiel 9, ersetzt aber das Pyrrolopyrrol der Formel IV mit der gleichen Menge des Pyrrrolopyrrols der Formel II (vgl. Beispiel 1) und das Pyrrolopyrrol der Formel V mit der gleichen Menge des Pyrrolopyrrols der Formel 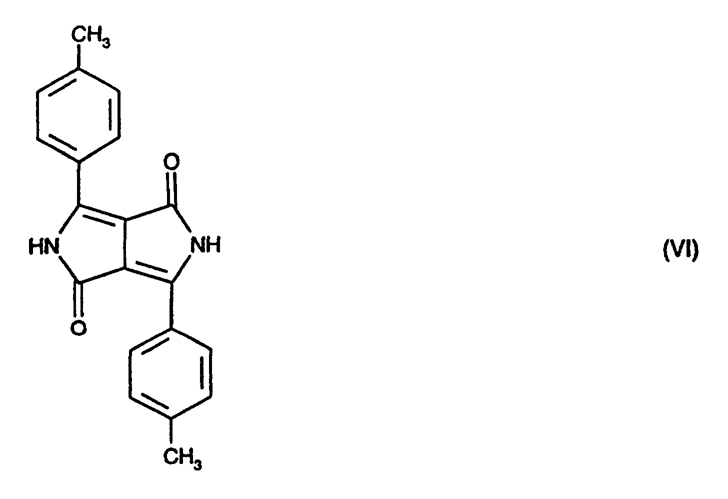 und führt den Spritzguss bei 280°C durch. Man erhält rote Platten der gleichen Farbe, wie diejenige der mit dem entsprechenden Mischkristall pigmentierten Platte.

Beispiel 12: Man verfährt wie in Beispiel 9, verwendet aber anstelle des Pyrrolopyrrols der Formel IV das Pyrrolopyrrol der Formel III (vgl. Beispiel 1) und anstelle des Pyrrolopyrrols der Formel V das Pyrrolopyrrol der Formel

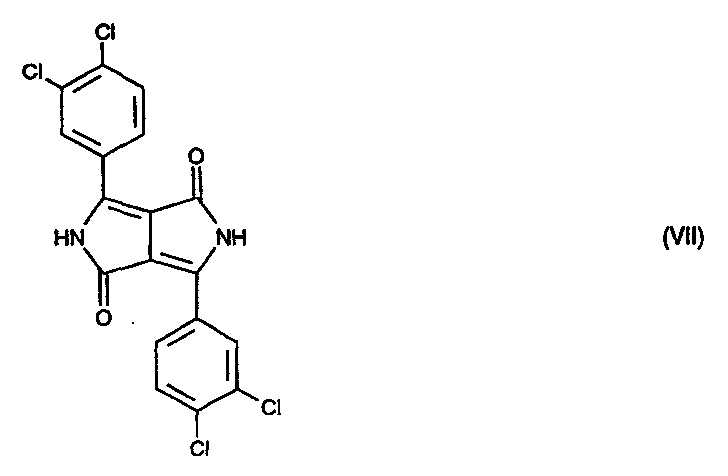

Man erhält hellrote Platten, deren Farbe derjenigen der Platten, die mit dem entsprechenden Mischkristall pigmentiert sind, entspricht.

## Patentansprüche

1. Verfahren zur Herstellung von Mischkristallen oder festen Lösungen von 1,4-Diketopyrrolo[3,4-c]pyrrolen, bestehend aus zwei unterschiedlichen Verbindungen der Formel 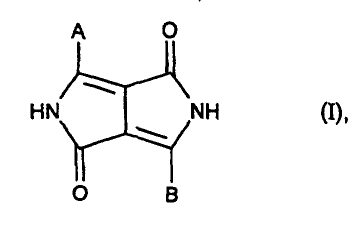
worin A und B, unabhängig voneinander für eine Gruppe der Formel 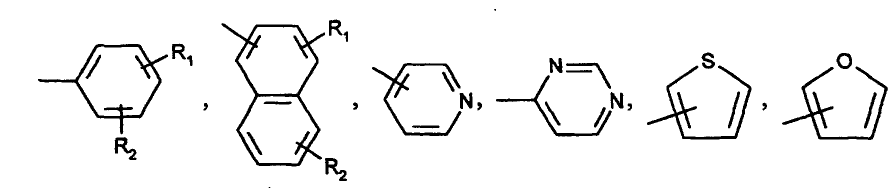 oder 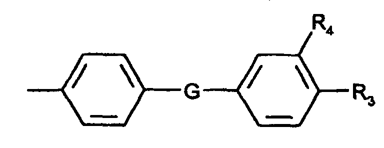 stehen, worin
R₁ und R₂ unabhängig voneinander Wasserstoff, Halogen, C₁-C₁₈-Alkyl, C₁-C₁₈-Alkoxy, C₁-C₁₈-Alkylmercapto, C₁-C₁₈-Alkylamino, C₂-C₁₈-Alkoxycarbonyl, C₂-C₁₈-Alkylaminocarbonyl, - CN, -NO₂, Phenyl, Trifluormethyl, C₅-C₆-Cycloalkyl, -C=N-(C₁-C₁₈-Alkyl), 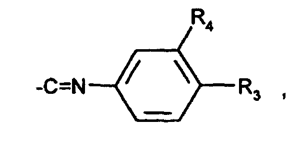 Imidazolyl, Pyrrazolyl, Triazolyl, Piperazinyl, Pyrrolyl, Oxazolyl, Benzoxazolyl, Benzthiazolyl, Benzimidazolyl, Morpholinyl, Piperidinyl oder Pyrrolidinyl bedeuten,
G -CH₂-, -CH(CH₃)-, -C(CH₃)₂-, -CH=N-, -N=N-, -O-, -S-, -SO-, -SO₂-, -CONH- oder -NR₇- ist,
R₃ und R ₄ unabhängig voneinander Wasserstoff, Halogen, C₁-C₆-Alkyl, C₁-C₁₈Alkoxy oder - CN sind, R₅ und R₆ unabhängig voneinander Wasserstoff, Halogen oder C₁-C₆-Alkyl und R₇ Wasserstoff oder C₁-C₆-Alkyl bedeuten, **dadurch gekennzeichnet, dass** ein Gemisch zweier unterschiedlicher Verbindungen der Formel I im Molverhältnis von 50:50 bis 95:5 in fester Form auf Temperaturen zwischen 220 und 380°C erhitzt wird.

2. Verfahren gemäss Anspruch 1, worin A und B unabhängig voneinander eine Gruppe der Formel 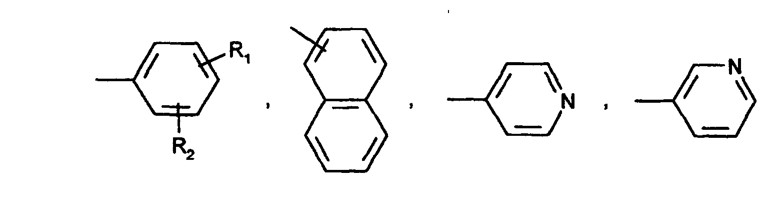 oder 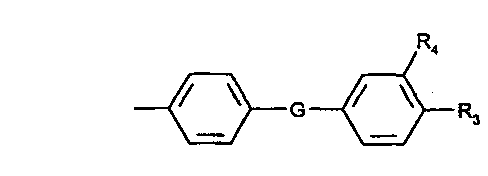 sind,
worin R₁ und R₂ unabhängig voneinander Wasserstoff, Chlor, Brom, C₁-C₄-Alkyl, C₁-C₆--Alkoxy, C₁-C₆-Alkylamino, Phenyl oder CN bedeuten,
G -O-, -NR₇-, -N=N- oder -SO₂- ist,
R₃ und R₄ Wasserstoff und R₇ Wasserstoff, Methyl oder Ethyl bedeuten.

3. Verfahren gemäss Anspruch 2, worin A und B gleich sind und eine Gruppe der Formel 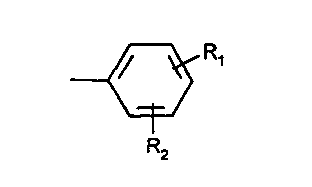 darstellen, worin R₁ und R₂ unabhängig voneinander Wasserstoff, Methyl, tert.-Butyl, Chlor, Brom, Phenyl oder CN bedeuten.

4. Verfahren gemäss Anspruch 3, worin R₂ Wasserstoff ist.

5. Verfahren gemäss Anspruch 1, worin die beiden unterschiedlichen Komponenten der Formel I auf Temperaturen zwischen 270 und 340°C erhitzt werden.

6. Verfahren gemäss Anspruch 1, worin die unterschiedlichen Komponenten der Formel I zusammen mit von 0 bis 20, vorzugsweise 0,5 bis 2 Gew%, bezogen auf die Komponentenmischung, eines Mischungshilfsmittels, innig vermischt werden.

7. Verfahren gemäss Anspruch 1, worin die unterschiedlichen Komponenten der Formel I nach allgemein üblichen Methoden innig vermischt werden und auf Temperaturen zwischen 220 und 380°C erhitzt werden.

8. Verfahren gemäss Anspruch 1, worin die unterschiedlichen Komponenten der Formel I nach allgemein üblichen Methoden innig vermischt werden und die so erhaltene Komponentenmischung in einem Sublimiergerät sublimiert wird.

9. Verfahren gemäss Anspruch 1, worin die unterschiedlichen Komponenten der Formel I einzeln abwechselnd in zwei oder mehreren dünnen Schichten im Hochvakuum auf ein geeignetes Substrat aufgedampft und mit einer Photopolymer-Schutzschicht überzogen werden und nachfolgend einer Laserbestrahlung unterzogen werden.

10. Verfahren gemäss Anspruch 1, worin zwei unterschiedliche Verbindungen der Formel I zusammen mit einem Polymer in einem Extruder bei Temperaturen von 160 bis 210°C dispergiert und anschliessend bei 220 bis 300°C zu Formen verspritzt werden.

11. Optisches Aufzeichnungsmaterial, erhältlich nach Anspruch 9.

## Claims

1. A process for the preparation of mixed crystals or solid solutions of 1,4-diketopyrrolo[3,4-c]pyrroles composed of two different compounds of formula 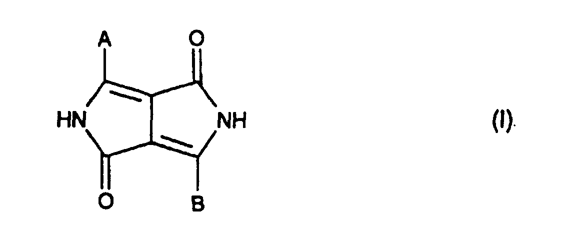 in which A and B are each independently of the her a group of formula 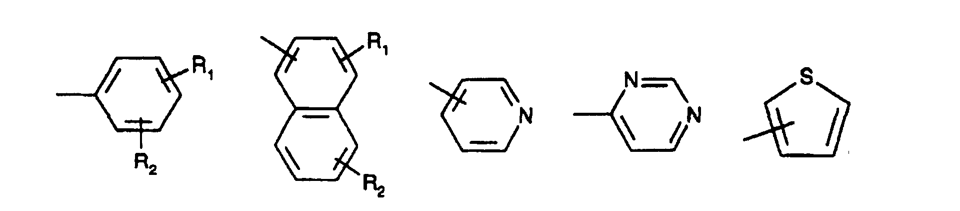 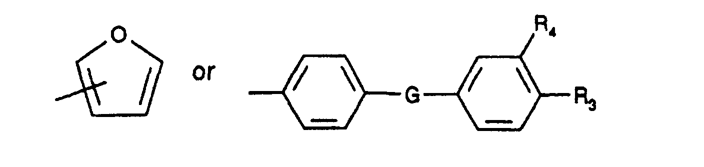 in which
R₁ and R₂ are each independently of the other hydrogen, halogen, C₁-C₁₈alkyl, C₁-C₁₈alkoxy, C₁-C₁₈alkylmercapto, C₁-C₁₈alkylamino, C₂-C₁₈alkoxycarbonyl, C₂-C₁₈alkylaminocarbonyl, -CN, -NO₂, phenyl, trifluoromethyl, C₅-C₆cycloalkyl, -C=N-(C₁-C₁₈alkyl), 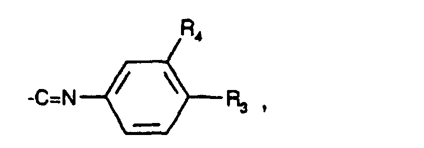 imidazolyl, pyrazolyl, triazolyl, piperazinyl, pyrrolyl, oxazolyl, benzoxazolyl, benzothiazolyl, benzimidazolyl, morpholinyl, piperidinyl or pyrrolidinyl,
G is -CH₂-, -CH(CH₃)-, -C(CH₃)₂-, -CH=N-, -N=N-, -O-, -S-, -SO-, -SO₂-, -CONH- or -NR₇-,
R₃ and R₄ are each independently of the other hydrogen, halogen, C₁-C₆alkyl, C₁-C₁₈alkoxy or -CN, R₅ and R₆ are each independently of the other hydrogen, halogen or C₁-C₆alkyl, and R₇ is hydrogen or C₁-C₆alkyl, which process comprises heating a mixture of two different compounds of formula I in a molar ratio of from 50:50 to 95:5 in solid form to temperatures of from 220 to 380°C.

2. A process according to claim 1, wherein A and B are each independently of the other a group of formula 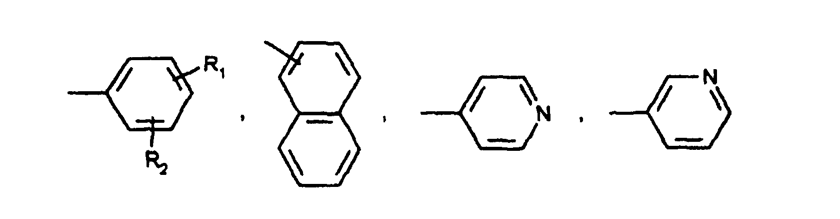 or 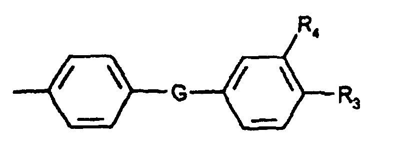
in which R₁ and R₂ are each independently of the other hydrogen, chloro, bromo, C₁-C₄alkyl, C₁-C₆alkoxy, C₁-C₆alkylamino, phenyl or CN,
G is -O-, -NR₇-, -N=N- or -SO₂-,
R₃ and R₄ are hydrogen, and R₇ is hydrogen, methyl or ethyl.

3. A process according to claim 2, wherein A and B are the same and are a group of formula 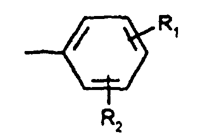 in which R₁ and R₂ are each independently of the other hydrogen, methyl, tert-butyl, chloro, bromo, phenyl or CN.

4. A process according to claim 3, wherein R₂ is hydrogen.

5. A process according to claim 1, wherein the two different components of formula I are heated to temperatures of from 270 to 340°C.

6. A process according to claim 1, which comprises intimately mixing the different components of formula I together with from 0 to 20%, preferably from 0.5 to 2%, by weight, based on the components mixture, of a mixing assistant.

7. A process according to claim 1, which comprises intimately mixing the different components of formula I by conventional methods and heating them to temperatures of from 220 to 380°C.

8. A process according to claim 1, which comprises intimately mixing the different components of formula I by conventional methods and sublimating the component mixture thus obtained in a sublimation apparatus.

9. A process according to claim 1, which comprises applying the different components of formula I by vapour deposition singly and alternately in two or more thin layers under high vacuum to a suitable substrate, coating them with a photopolymer protective layer and subsequently subjecting them to laser irradiation.

10. Process according to claim 1, which comprises dispersing two different compounds of formula I together with a polymer in an extruder at temperatures of from 160 to 210°C and then injection-moulding them to shapes at from 220 to 300°C.

11. An optical recording material obtainable according to claim 9.

## Revendications

1. Procédé pour la préparation de cristaux mixtes ou de solutions solides de 1,4-dicétopyrrolo[3,4-c]pyrroles, constitués par deux composés différents de formule 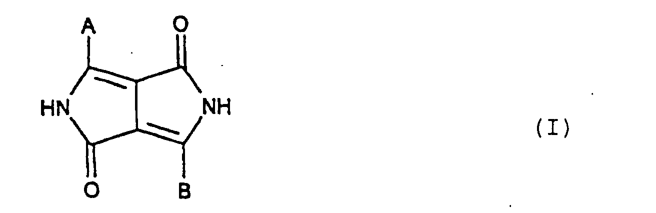 où A et B représentent, indépendamment l'un de l'autre, un groupe de formule 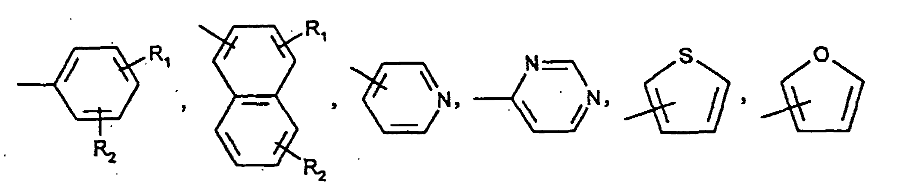 ou 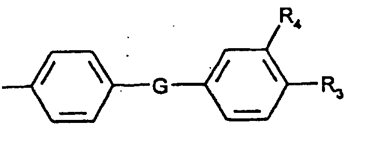 où
R₁ et R₂ représentent, indépendamment l'un de l'autre, des atomes d'hydrogène, des atomes d'halogènes, des groupes alkyle en C₁-C₁₈, alkoxy en C₁-C₁₈, (alkyl en C₁-C₁₈)mercapto, (alkyl en C₁-C₁₈)amino, (alkoxy en C₂-C₁₈) carbonyle, (alkyl en C₂-C₁₈)-aminocarbonyle, -CN, -NO₂, phényle, trifluorométhyle, cycloalkyle en C₅-C₆, -C=N-(alkyle en C₁-C₁₈), 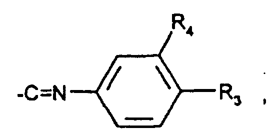 imidazolyle, pyrrazolyle, triazolyle, pipérazinyle, pyrrolyle, oxazolyle, benzoxazolyle, benzthiazolyle, benzimidazolyle, morpholinyle, pipéridinyle ou pyrrolidinyle,
G représente un groupe -CH₂-, -CH(CH₃)-, -C(CH₃)₂-, -CH=N-, -N=N-, -O-, -S-, -SO-, -SO₂-, -CONH- ou NR₇-,
R₃ et R₄ représentent, indépendamment l'un de l'autre, des atomes d'hydrogène, des atomes d'halogènes, des groupes alkyle en C₁-C₆, alkoxy en C₁-C₁₈ ou -CN, R₅ et
R₆ représentent, indépendamment l'un de l'autre, des atomes d'hydrogène, des atomes d'halogènes ou des groupes alkyle en C₁-C₆, R₇ représente un atome d'hydrogène ou un groupe alkyle C₁-C₆, **caractérisé en ce qu'**on chauffe un mélange de deux composés différents de formule I dans un rapport molaire de 50:50 à 95:5 sous forme solide à une température comprise entre 220 et 380°C.

2. Procédé selon la revendication 1, où A et B représentent, indépendamment l'un de l'autre, un groupe de formule 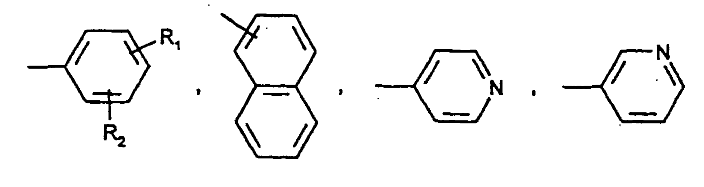 ou 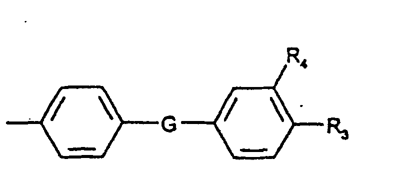 où
R₁ et R₂ représentent, indépendamment l'un de l'autre, des atomes d'hydrogène, de chlore, de brome, des groupes alkyle en C₁-C₄, alkoxy en C₁-C₆, (alkyl en C₁-C₆)amino, phényle ou CN,
G représente un groupe -O-,- NR₇-, -N=N- ou -SO₂,
R₃ et R₄ représentent un atome d'hydrogène et R₇ représente un atome d'hydrogène, des groupes méthyle ou éthyle.

3. Procédé selon la revendication 2, où A et B sont identiques et représentent un groupe de formule 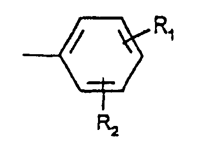 où R₁ et R₂ représentent, indépendamment l'un de l'autre, un atome d'hydrogène, des groupes méthyle, tert.-butyle, chloro, bromo, phényle ou CN.

4. Procédé selon la revendication 3, où R₂ représente un atome d'hydrogène.

5. Procédé selon la revendication 1, où l'on chauffe les deux composants différents de formule I à des températures comprises entre 270 et 340°C.

6. Procédé selon la revendication 1, où l'on mélange intimement les différents composants de formule I avec 0 à 20, de préférence de 0,5 à 2 % en masse, par rapport au mélange de composants, d'un adjuvant de mélange.

7. Procédé selon la revendication 1, où l'on mélange intimement les composants de formule I différents selon des méthodes généralement usuelles et l'on chauffe à des températures comprises entre 220 et 380°C.

8. Procédé selon la revendication 1, où l'on mélange intimement les composants de formule I différents selon des méthodes généralement usuelles et l'on sublime le mélange de composants ainsi obtenu dans un appareil de sublimation.

9. Procédé selon la revendication 1, où l'on dépose les composants de formule I différents seuls, alternativement dans deux ou plusieurs couches minces, sous vide poussé sur un substrat approprié et et l'on revêt d'une couche protectrice photopolymère et ensuite on soumet à une irradiation au laser.

10. Procédé selon la revendication 1, où l'on disperse deux composés de formule I différents conjointement avec un polymère dans une extrudeuse à des températures de 160 à 210°C et ensuite l'on injecte dans un moule à une température de 220 à 300°C.

11. Matière d'enregistrement optique, que l'on peut obtenir selon la revendication 9.
